(19) Europäisches Patentamt
European Patent Office
Office européen des brevets

(11) Numéro de publication: **0 240 424 B1**

(12) # FASCICULE DE BREVET EUROPEEN

(45) Date de publication de fascicule du brevet: **29.05.91**

(51) Int. Cl.[5]: **A61K 9/50**

(21) Numéro de dépôt: **87400695.0**

(22) Date de dépôt: **27.03.87**

(54) **Nanoparticules à base de polymère ou copolymère méthacrylique, procédé de préparation, et application comme vecteur de médicament.**

(30) Priorité: **28.03.86 FR 8604535**

(43) Date de publication de la demande: **07.10.87 Bulletin 87/41**

(45) Mention de la délivrance du brevet: **29.05.91 Bulletin 91/22**

(84) Etats contractants désignés: **BE CH DE ES FR GB IT LI LU NL**

(56) Documents cités:
**FR-A- 2 304 326**

**CHEMICAL ABSTRACTS, vol. 98, no. 22, mai 1983, page 364, résumé no. 185458v, Columbus, Ohio, US; J. KREUTER: "Physicochemical characterization of polyacrylic nanoparticles", & INT. J. PHARM 1983, 14(1), 43-58**

**JOURNAL OF PHARMACEUTICAL SCIENCES, vol. 65, no. 12, décembre 1976, pages 1763-1766; G. BIRRENBACH et al.: "Polymerized micelles and their use as adjuvants in immunology"**

**CHEMICAL ABSTRACTS, vol. 104, no. 12, 24 mars 1986, pages 438,439, résumé no. 95395g, Columbus, Ohio, US; S.J. DOUGLAS et al.: "Poly(butyl 2-cyanoacrylate) nanoparticles with differing surface charges", & J. CONTROLLED RELEASE 1986, 3(1), 15-23**

(73) Titulaire: **UNIVERSITE DE RENNES I**
**2, rue du Thabor**
**F-35000 Rennes(FR)**

(72) Inventeur: **Leverge, Roger**
**5, rue de Robien**
**F-35000 Rennes(FR)**
Inventeur: **Rolland, Alain**
**9, cours Kennedy**
**F-35000 Rennes(FR)**

(74) Mandataire: **L'Helgoualch, Jean**
**Cabinet Sueur et L'Helgoualch 78, rue Carnot**
**F-95240 Cormeilles-en-Parisis(FR)**

**EP 0 240 424 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne de nouvelles nanoparticules formées par polymérisation de monomères méthacryliques, leur application en thérapeutique comme vecteurs de médicaments et comme bioréactifs, ainsi qu'un procédé pour leur préparation.

Les tentatives d'optimisation des performances thérapeutiques des médicaments ont abouti à la conception de nouvelles formes galéniques représentées notamment par les "systèmes vecteurs" qui ont fait l'objet d'importants travaux de recherche depuis quelques années. Une molécule pharmacologiquement active pilotée par un vecteur médicamenteux peut être dirigée dans l'organisme vers les "cellules cibles" à traiter où elle est alors concentrée de façon importante, ce qui permet d'améliorer l'efficacité thérapeutique tout en diminuant la dose initiale à administrer. De plus, la limitation du champ d'activité au site choisi évite l'imprégnation d'autres organes, et, par conséquent, l'apparition d'effets secondaires indésirables.

Les vecteurs de médicaments sont généralement constitués de particules qui doivent pouvoir former une liaison stable avec la molécule pharmacologiquement active pour éviter sa libération prématurée dans l'organisme, mais cette liaison doit être réversible pour que la molécule soit libérée au niveau du site d'action. La fixation des diverses molécules actives est liée à la nature hydrophile ou lipophile de la particule, de même que sa distribution dans l'organisme, ainsi que la possibilité de couplage avec des anticorps monoclonaux.

On sait également que la taille des particules est un paramètre important qui régit sa distribution tissulaire ou cellulaire et qui conditionne sa tolérance pour une administration intravasculaire, et on a constaté qu'une taille de particule inférieure au $\mu$m est nécessaire.

On a notamment proposé d'utiliser des nanoparticules de polymères comme vecteurs de médicaments. Par "nanoparticules" on entend des particules colloïdales de taille variant entre 10 et 1.000nm dans lesquelles les molécules actives sont dissoutes, entrappées, ou encapsulées, ou sur lesquelles elles sont adsorbées ou fixées.

Selon les techniques de polymérisation mises en oeuvre pour leur préparation, il est possible d'obtenir des colloïdes creux (nanocapsules) ou des particules compactes de polymère (nanoparticules). Il est possible également, en fonction des monomères initiaux utilisés, de faire varier la nature du réseau polymérique et d'en modifier la porosité, la dégradabilité et la biodisponibilité.

De tels vecteurs trouvent des applications dans de nombreux domaines. Ils ont été utilisés comme adjuvants immunologiques, comme supports d'hormones, d'enzymes, de cytostatiques, etc. La majorité des formulations nanoparticulaires mises au point récemment est de nature acrylique. Par rapport aux autres vecteurs médicamenteux, ces nanoparticules présentent l'avantage d'une bonne tolérance par voie parentérale. De plus, leur formulation est généralement stable et reproductible. Toutefois, la plupart des nanoparticules acryliques ont l'inconvénient de présenter une biodégradabilité lente des polymères utilisés, d'où la possibilité d'une accumulation au niveau intracellulaire, notamment en ce qui concerne les organes appartenant au système réticuloendothélial.

La demande de brevet européen 0.064.967 décrit des nanoparticules biodégradables de diamètre inférieur à 600nm préparées par polymérisation d'un cyanoacrylate d'alkyle dans un milieu aqueux en absence d'agent tensio-actif. Toutefois, les vecteurs de médicaments ainsi préparés ont tendance à libérer prématurément le principe actif adsorbé en raison des interactions avec les protéines du plasma sanguin. De plus ces vecteurs ne comprennent pas en surface de groupe réactif facilitant la fixation de molécules.

Des nanoparticules à base de cyanoacrylate, stabilisées par un dérivé de dextrane, sont également décrites par D. Stephen et al. J. Controlled Release (1986) 3(1) 15-23.

Un procédé de polymérisation micellaire permettant de former des nanoparticules dans lesquelles sont entrappées des substances biologiquement actives, est décrit par G. Birrenbach et al. J. of Pharm. Sciences vol 65 n°12 (1976).

Le brevet Français FR 2.304.326 décrit des particules submicroscopiques sur lesquelles peuvent être adsorbées des substances biologiquement actives. Ces particules peuvent être préparées par polymérisation de divers monomères, tels que des monomères acryliques ou méthacryliques, mais, selon ce brevet, les acides acrylique et méthacrylique eux-mêmes doivent être exclus en raison de leur fonction acide. L'adsorption des substances est cependant limitée et réversible, et ne résiste pas au déplacement de la particule dans un milieu biologique. L'efficacité de ces particules comme vecteurs de médicaments est donc insuffisante. Des propriétés physico-chimiques de telles nanoparticules sont décrites dans Chem. Abst. Vol.98, n° 195458v (1983).

On a constaté au contraire, suivant la présente invention, que des vecteurs de médicaments efficaces peuvent être obtenus au moyen de nanoparticules à base de polymères ou copolymères méthacryliques portant des groupes acide carboxylique et/ou hydroxyle en surface ou au voisinage de la surface de la

particule.

La présente invention a pour objet de nouvelles nanoparticules à base de polymères ou copolymères méthacryliques ainsi que leur application en thérapeutique comme vecteurs de médicaments, produits de contraste en imagerie médicale, ou bioréactifs.

L'invention a plus particulièrement pour objet de nouvelles nanoparticules à base de polymères ou copolymères méthacryliques de taille homogène et inférieure à 600nm, de caractère hydrophile/lipophile variable, susceptibles de fixer des molécules pharmacologiquement actives ou bioréactives par adsorption ou par liaison chimique.

L'invention a également pour objet un procédé de préparation de nanoparticules à base de polymères ou copolymères méthacryliques, procurant une distribution homogène monodisperse de la taille des nanoparticules.

Enfin, l'invention a pour objet les nouveaux vecteurs de médicaments et les réactifs de diagnostic médical ainsi obtenus.

Les nanoparticules suivant la présente invention, utilisables comme vecteurs de médicaments, sont des particules de polymère à base de méthacrylate d'alkyle ou de copolymère à base de méthacrylate d'alkyle et d'un autre monomère copolymérisable avec lui, de forme sphérique, de diamètre moyen compris entre 100 et 600nm, et de préférence entre 200 et 400nm, de distribution homogène (système monodispersé), présentant en surface ou au voisinage de la surface des groupements réactionnels hydroxyle et/ou carboxyle.

Plus particulièrement, les nanoparticules conformes à la présente invention sont à base de polymère ou copolymère comprenant au moins une unité récurrente de formule générale (I) ci-après:

$$\begin{matrix} & COOR & \\ & | & \\ -CH- & C & - \\ & | & \\ & CH_3 & \end{matrix}$$

dans laquelle R représente un atome d'hydrogène ou un groupe alkyle linéaire ou ramifié de 1 à 6 atomes de carbone, ou un groupe alkyle substitué par un groupe hydroxy, carboxy ou ester, comportant le cas échéant une ou plusieurs doubles liaisons éthyléniques, l'une au moins des unités récurrentes comportant au moins un groupe carboxyle et/ou hydroxyle libre.

Les nanoparticules suivant la présente invention sont préparées par polymérisation en émulsion à partir d'au moins un monomère méthacrylique de formule générale (II) ci-après:

$$\begin{matrix} CH_2 = & C & - COOR \\ & | & \\ & CH_3 & \end{matrix}$$

dans laquelle R représente un atome d'hydrogène ou un groupe alkyle linéaire ou ramifié de 1 à 6 atomes de carbone, ou un groupe alkyle substitué par un groupe hydroxy, carboxy ou ester, comportant le cas échéant une ou plusieurs doubles liaisons éthyléniques, l'un au moins des monomères comportant un groupe R alkyle substitué par un groupe hydroxy et/ou carboxy et/ou ester.

Comme indiqué ci-dessus, la polymérisation peut s'effectuer de la même manière à partir de plusieurs monomères copolymérisables entre eux.

Dans la formule générale (I) ci-dessus, R peut représenter notamment un atome d'hydrogène lorsque le monomère de base est l'acide méthacrylique, ou un groupe alkyle choisi parmi les groupes méthyle, éthyle, isopropyle, n-butyle, t-butyle, etc, un groupe alkyle substitué par un groupe hydroxy, tel qu'un groupe hydroxy-2 propyle ou dihydroxy-2,4 butyle, ou encore un groupe isopropényl carboxylate. Ainsi, à titre d'exemple, outre l'acide méthacrylique, on peut utiliser comme monomère le méthacrylate de méthyle ou de n-butyle, le méthacrylate d'hydroxy-2 propyle ou le diméthacrylate d'éthylène glycol. Le diméthacrylate d'éthylène glycol, utilisé comme comonomère, présente l'avantage de renforcer la stabilité du produit polymérisé en raison de son action comme agent de réticulation. Ces monomères peuvent être utilisés isolément pour former des homopolymères, ou en combinaison de deux ou plusieurs copolymérisables entre eux pour former des copolymères.

La réaction de polymérisation en émulsion s'effectue en phase aqueuse, en présence d'un initiateur de radicaux libres, sous agitation, à une température comprise entre 60 et 120 ° C, et de préférence entre 80 et 90 ° C, pendant une durée comprise entre 30 minutes environ et quelques heures, selon les monomères et additifs utilisés. Les conditions de la réaction de polymérisation peuvent être adaptées selon les circonstances conformément aux techniques usuelles. En particulier, il peut être avantageux d'ajouter au milieu réactionnel un agent tensio-actif qui favorise une diminution et une uniformisation de la taille des nanoparticules.

Par exemple, le ou les monomères sont ajoutés sous agitation mécanique dans une phase aqueuse contenant un initiateur de radicaux libres, et le cas échéant un agent tensio-actif, et la réaction s'effectue à une température comprise entre 80 et 90 ° C pendant une heure environ. On obtient ainsi une suspension laiteuse de nanoparticules conformes à la présente invention.

Le nombre de nanoparticules formées suivant le procédé de l'invention est fonction de l'initiateur de radicaux libres, de l'agent tensio-actif présent le cas échéant, et du ou des monomères utilisés, et en particulier de leurs concentrations respectives, ainsi que de la température réactionnelle.

L'initiateur de radicaux libres peut être choisi parmi les composés usuels de la technique de polymérisation en émulsion, et par exemple on peut utiliser le peroxodisulfate de potassium, le persulfate d'ammonium ou le persulfate de potassium, dans le cas de l'initiation chimique. La concentration de l'initiateur de radicaux libres, exprimée par rapport au volume total de la solution réactionnelle, doit être comprise de préférence entre 0,01 et 0,2%, et par exemple, dans le cas de la polymérisation du méthacrylate de méthyle, pour une réaction s'effectuant à 80 ° C pendant une heure environ, la teneur en peroxodisulfate de potassium pourra être avantageusement comprise entre 0,01 et 0,05%. Si la concentration est trop faible, on observe la formation d'agglo-mérats polymérisés résiduels. Une concentration plus élevée que la valeur indiquée ci-dessus n'a plus d'influence sur la taille des nanoparticules. Suivant une variante utilisable dans la présente invention, l'initiation radicalaire peut s'effectuer par irradiation, par exemple par des rayons gamma.

L'agent tensio-actif utilisé le cas échéant est dissous au moins en partie dans la phase aqueuse contenant le ou les monomères à polymériser. Il est préférable d'utiliser un agent tensio-actif aussi peu toxique que possible, en évitant par conséquent l'utilisation d'agents tensio-actifs ioniques dont la toxicité est parfois non négligeable. On peut utiliser notamment un agent tensioactif tel que le Pluriol PE 6800 (BASF) résultant de la copolymérisation de l'oxyde de propylène et de l'oxyde d'éthylène. La teneur en agent tensio-actif exerce une influence sur la taille des nanoparticules. Plus cette teneur est élevée, plus la taille des particules a tendance à diminuer. Toutefois, une teneur trop élevée en agent tensio-actif peut entraîner une plus grande dispersion de la distribution des tailles des nanoparticules formées. Pour cette raison, il est préférable que la teneur en agent tensio-actif soit inférieure à 0,5% environ par rapport au volume total du milieu réactionnel.

Le ou les monomères méthacryliques utilisés dans l'invention sont des composés connus, disponibles dans le commerce. Compte-tenu de l'application envisagée dans le domaine médical, il est préférable de les purifier au préalable. La purification peut être faite par les procédés usuels de la technique, et par exemple par distillation sous vide partiel, à l'abri de la lumière, de préférence sous atmosphère inerte.

La concentration du ou des monomères à polymériser est choisie selon les conditions de la réaction et les résultats recherchés. Il est préférable que cette concentration soit inférieure à 10% environ, et généralement elle doit être inférieure à 6%. Au-delà de cette valeur, la taille des particules devient plus importante et on observe la formation d'un résidu important de polymères agglomérés. Inversement, une concentration trop faible procure des particules dont la distribution des tailles est trop dispersée. On pourra avantageusement utiliser un ou plusieurs monomères en une concentration comprise entre 1 et 6% pour obtenir, par une polymérisation à 90 ° C pendant une heure environ, des nanoparticules de diamètre compris entre 200 et 400nm.

La taille des nanoparticules obtenues conformément à la présente invention dépend bien entendu de la nature des monomères utilisés dans la réaction de polymérisation. Ainsi, quand on fait évoluer les pourcentages relatifs des divers monomères à copolymériser, les autres conditions de la réaction restant constantes, on constate qu'une augmentation de la teneur en méthacrylate d'hydroxypropyle a tendance à provoquer une élévation de la taille des nanoparticules obtenues. On peut mettre à profit cette particularité pour modifier la taille des nanoparticules entre 200 et 500nm.

La température de polymérisation est choisie en fonction des monomères et des additifs utilisés. Comme indiqué ci-dessus, elle peut être comprise entre 60 et 120 ° C, et de préférence entre 80 et 90 ° C environ. Une élévation de la température de polymérisation entraîne une diminution de la taille des nanoparticules. Ainsi par exemple, dans le cas de la polymérisation du méthacrylate d'hydroxypropyle, pour une polymérisation de 100ml d'une solution aqueuse à 5% de monomère en présence de 10mg de

persulfate de potassium comme initiateur de radicaux libres, pendant une heure environ, la taille des nanoparticules diminue de 520nm à 360nm environ lorsque la température passe de 80 à 90 °C.

Les nanoparticules ainsi obtenues peuvent être purifiées par les techniques connues. Il peut être par exemple avantageux d'éliminer les monomères non polymérisés et les initiateurs de radicaux libres résiduels par une méthode de dialyse dynamique. Cette purification permet de stabiliser la suspension des nanoparticules et d'éliminer les risques de toxicité qui pourraient résulter de la présence de monomères résiduels ou d'initiateurs de radicaux libres, lors de l'administration des nanoparticules par voie intravasculaire. Le contrôle de la pureté peut s'effectuer par chromatographie liquide haute performance en phase inverse. Ce contrôle ne fait apparaître aucune trace des divers monomères utilisés pour la réaction de polymérisation, ce qui démontre la bonne efficacité de la méthode de purification par dialyse.

L'analyse morphométrique des nanoparticules conformes à la présente invention peut être effectuée au moyen d'un compteur à diffraction du type Nanosizer (Coultronics). Cette analyse montre que le diamètre moyen des nanoparticules est compris entre 100 et 600nm selon les polymères ou copolymères préparés. L'examen au microscope électronique à balayage montre que les nanoparticules se présentent sous la forme de billes homogènes sphériques, non agglomérées.

L'analyse des nanoparticules conformes à la présente invention par spectroscopie infra-rouge et de RMN met en évidence la présence de groupes fonctionnels carboxyliques et/ou hydroxyles libres en surface. Ainsi, le spectre de RMN d'une solution dans le diméthylsulfoxyde de nanoparticules en copolymère acide méthacrylique - méthacrylate de méthyle - méthacrylate d'hydroxypropyle, fait apparaître un pic à 12,45ppm caractéristique de la présence de groupes COOH. De même, le spectre infra-rouge en pastille de KBr révèle la présence de groupes COOH et/ou OH libres.

Le dosage titrimétrique à l'aide de solution d'hydroxyde de sodium en présence de chlorure de potassium montre que les nanoparticules de l'invention comportent à leur surface ou dans son voisinage un nombre élevé de groupes réactionnels, pouvant être de l'ordre de $10^4$ à $10^7$ groupes COOH par particule. De plus, en raison de la porosité du polymère ou du copolymère, non seulement les groupes situés en surface, mais aussi les groupes se trouvant au voisinage de la surface, sont accessibles et permettent la formation de liaisons covalentes avec diverses substances, telles que des substances biologiquement actives.

La masse moléculaire des polymères et copolymères peut être évaluée par chromatographie par perméation de gel, suivant une méthode usuelle, en utilisant le tétrahydrofuranne comme solvant, par référence à des échantillons standards de polystyrène. On constate ainsi que les nanoparticules obtenues conformément à la présente invention présentent des masses moléculaires relativement importantes, comprises entre 200.000 et 600.000 environ.

Comme indiqué ci-dessus, les nanoparticules conformes à la présente invention peuvent être utilisées comme vecteurs de médicaments, les principes actifs étant fixés sur les nanoparticules par adsorption et/ou par liaison chimique covalente. La fixation peut être simplement réalisée en ajoutant une solution concentrée du principe actif dans un solvant approprié, par exemple de l'eau distillée, à une suspension de nanoparticules suivant l'invention, dans un rapport volumique compris entre 1/20 et 3/10 environ, à un pH de préférence compris entre 6 et 8, et en maintenant le milieu sous agitation à température ambiante, ou en chauffant à une température comprise entre 20 et 60 °C, pendant 30 minutes à 3 heures environ. Suivant une variante conforme à la présente invention, il est possible d'effectuer le couplage du principe actif avec les nanoparticules en ajoutant une solution du principe actif à une suspension lyophilisée de nanoparticules.

Des mesures de la capacité d'adsorption ont été faites sur des suspensions de nanoparticules purifiées par dialyse, comme indiqué ci-dessus, pendant 24 heures environ, puis ajustées à un pH voisin de 8 par addition d'hydroxyde de sodium et d'acétate de sodium, pour favoriser la fixation. Les mesures ont été effectuées après fixation de principes actifs ou réactifs tels que la doxorubicine, la bléomycine, la daunorubicine, le chlorure de manganèse, l'orange acridine et le bromure d'éthidium. Le taux d'adsorption ainsi mesuré est supérieur à 95% pour toutes les nanoparticules de l'invention, et égal à 100% pour la plupart d'entre elles.

Ces résultats montrent que les nanoparticules conformes à la présente invention permettent la fixation de diverses molécules pharmacologiquement actives, de réactifs de diagnostic, d'agents de contraste utilisables en imagerie médicale, ou d'anticorps monoclonaux, la fixation étant renforcée par les liaisons covalentes formées entre les groupes carboxyles et/ou hydroxyles, à la surface des nanoparticules, et les molécules. Il est en particulier possible de préparer des composés immunoréactifs constitués par des nanoparticules suivant la présente invention sur lesquelles sont fixés des composés fluorescents et des anticorps monoclonaux.

Suivant une forme particulière de mise en oeuvre de la présente invention, on forme une liaison covalente entre les nanoparticules et un anticorps monoclonal, par l'intermédiaire d'un "bras espaceur"

dérivé par exemple d'un composé de type diaminoalcane comportant deux groupes amino séparés par une chaîne alkylène de 4 à 10 atomes de carbone, tel qu'un diaminoheptane ou diaminohexane, que l'on fixe aux groupes carboxyles libres en surface des nanoparticules de l'invention, d'une part, par liaison covalente, par réaction de couplage, par exemple en présence de carbodiimide, puis aux groupes carboxyles des anticorps, d'autre part, de préférence après activation par le glutaraldéhyde. Il est bien entendu possible de lier directement les groupes acide carboxylique en surface des nanoparticules avec les restes $-NH_2$ des anticorps, pour former par covalence une liaison amide, par exemple en présence de carbodiimide, suivant une technique connue.

Ce mode de liaison entre les nanoparticules et les anticorps monoclonaux procure une stabilité optimale du système formé: au contraire, une simple adsorption présente l'inconvénient d'être réversible, et l'équilibre est déplacé dans le sens d'une désorption des anticorps monoclonaux par divers composants sanguins possédant une affinité pour les nanoparticules.

L'efficacité du couplage d'anticorps monoclonaux aux nanoparticules suivant la présente invention pour former des nanovecteurs biologiques peut être démontrée au moyen d'une modèle cellulaire comprenant un mélange de deux populations de lymphocytes (B et T) permettant de visualiser le ciblage spécifique des nanoparticules couplées à des anticorps antilymphocytes T sur les seuls lymphocytes T. Outre les anticorps antilymphocytes T, on peut utiliser par exemple les anticorps anticellule du cancer épidermoïde du cancer, ou anticellule métastasique des cancers rectocoliques.

De plus, la présente invention possède l'avantage de permettre la conservation des vecteurs constitués par les nanoparticules en suspension, sur lesquelles peuvent être fixés les principes actifs ou les bioréactifs, par simple lyophilisation. Un autre avantage de la présente invention est de permettre la conservation des suspensions de nanoparticules, prêtes à l'emploi et au couplage avec des molécules actives, en flacons stériles, après purification et stérilisation à l'autoclave à une température légèrement supérieure à 100° C, par exemple à 105° C.

Comme indiqué ci-dessus, les nanoparticules conformes à la présente invention peuvent également être utilisées en imagerie médicale, et plus particulièrement en imagerie médicale par résonance magnétique nucléaire (RMN). Suivant l'invention, des produits connus, permettant ou améliorant l'obtention d'une image par RMN, comme par exemple des sels de manganèse, notamment le chlorure de manganèse, des oxydes de fer, notamment la magnétite, ainsi que des dérivés de gadolinium, tels que des complexes organiques de gadolinium utilisés en RMN, sont fixés ou adsorbés sur les nanoparticules. En particulier, on peut incorporer des oxydes de fer en cours de polymérisation, et ces produits peuvent alors agir comme initiateurs de radicaux libres pour la réaction de polymérisation, puis comme agents de vectorisation des nanoparticules finies, sous l'influence d'un champ magnétique, dans la méthode d'imagerie par RMN.

La préparation de divers polymères et copolymères méthacryliques, conformément à la présente invention, est décrite ci-après pour illustrer l'invention plus en détail.

EXEMPLE 1

Dans 95ml d'une solution aqueuse à 0,001% de Pluriol PE 6800 (BASF) contenant 10mg de persulfate de potassium, on verse 5ml de méthacrylate de méthyle purifié par distillation sous vide partiel à 22° C, à l'abri de la lumière, sous atmosphère d'azote, en présence d'hydroquinone.

Le milieu est maintenu sous agitation mécanique, et on chauffe à 90° C pendant une heure pour provoquer la polymérisation.

On obtient ainsi une suspension laiteuse de nanoparticules de polyméthacrylate de méthyle, que l'on filtre sur verre fritté de porosité 10 à 20 $\mu$m afin d'éliminer les aggrégats éventuels.

Le diamètre moyen des nanoparticules mesuré au moyen d'un compteur à diffraction Nanosizer (Coultronics) est de 250nm et l'indice de dispersion est égal à 0 (système monodisperse). Le poids moléculaire moyen, mesuré par la méthode de chromatographie par perméation de gel, est de 540.000 environ.

En répétant le même procédé mais avec 50mg de persulfate de potassium comme initiateur de radicaux libres, on obtient les mêmes résultats. Par contre, en utilisant 5mg de persulfate de potassium, le diamètre moyen des nanoparticules obtenues est de 200nm seulement, mais on observe la formation d'agglomérats polymérisés.

Pour évaluer l'incidence de la teneur en agent tensio-actif sur la taille des particules, on effectue quatre essais supplémentaires en utilisant respectivement une solution aqueuse de Pluriol à 0,01% (essai A), 0,1% (essai B), 0,5% (essai C) tandis que dans l'essai D aucun agent tensio-actif n'est utilisé. Dans chacun de ces essais, la quantité de méthacrylate de méthyle utilisée est de 5ml. Les résultats sont indiqués ci-

dessous.

| Essai | A | B | C | D |
|---|---|---|---|---|
| Taille | 217 | 136 | 92 | 202 |
| Dispersion | 0 | 2 | 4 | 2 |

Dans le tableau ci-dessus, la taille des nanoparticules est exprimée en nanomètres. Un faible indice de dispersion (0 à 2) correspond à un système monodisperse.

EXEMPLE 2

On procède comme dans l'exemple 1, en remplaçant le méthacrylate de méthyle par une même quantité de méthacrylate d'hydroxy-2 propyle.

On obtient ainsi des nanoparticules de polyméthacrylate d'hydroxy-2 propyle, de diamètre moyen égal à 365nm et d'indice de dispersion égal à 0.

En procédant de la même manière, mais en utilisant 2ml de monomère, les autres conditions restant inchangées, on obtient des nanoparticules de diamètre moyen égal à 278nm et d'indice de dispersion égal à 1.

Pour évaluer l'incidence de la température de polymérisation sur les nanoparticules obtenues, on effectue deux essais supplémentaires au moyen de 5ml du même nanomère que ci-dessus, dans les mêmes conditions, mais en chauffant à 80°C dans le premier cas, et à 85°C dans le deuxième. On observe que le diamètre moyen des nanoparticules obtenues est égal à 522nm dans le premier cas, et 411nm dans le deuxième, l'indice de dispersion n'étant pas sensiblement modifié.

EXEMPLE 3

On procède comme dans l'exemple 1 mais en remplaçant le monomère par une même quantité d'un mélange de plusieurs monomères, afin de préparer un copolymère méthacrylique.

Les résultats sont indiqués au tableau ci-après qui fait apparaître pour chaque copolymère la composition en monomères (pourcentage volumique) et le diamètre moyen des nanoparticules (nanomètres).

| | Composition | | | | |
|---|---|---|---|---|---|
| Copol. | MMA | HPM | AM | EGD | Taille |
| A | 56 | 30 | 10 | 4 | 311 |
| B | 60 | 30 | 10 | 0 | 299 |
| C | 56 | 40 | 0 | 4 | 282 |
| D | 0 | 86 | 10 | 4 | 472 |
| E | 6 | 80 | 10 | 4 | 416 |
| F | 42 | 42 | 12 | 4 | 385 |
| G | 0 | 90 | 10 | 0 | 477 |

Dans le tableau ci-dessus, les monomères utilisés sont désignés par les abréviations suivantes. MMA: Méthacrylate de méthyle. HPM: Méthacrylate d'hydroxy-2 propyle. AM: Acide méthacrylique. EGD: Diméthacrylate d'éthylène glycol.

EXEMPLE 4

On utilise les nanoparticules ayant la composition A indiquée dans l'exemple 3, après filtration de la suspension nanoparticulaire sur verre fritté (porosité: 10-20 $\mu$m) puis purification par dialyse dynamique. Le milieu est tamponné à pH 8,0 par addition d'acétate de sodium 0,01M et hydroxyde de sodium N.

On réalise le couplage de ces nanoparticules avec la doxorubicine, en ajoutant une solution concentrée de chlorhydrate de doxorubicine dans de l'eau distillée, à la suspension de nanoparticules et en maintenant le milieu à température ambiante pendant 2 heures sous agitation.

L'étude de la cytotoxicité de la doxorubicine sur des cellules en culture (lignée Blastoïde U937) montre que la cytotoxicité des nanoparticules couplées à la doxorubicine est plus de 10 fois supérieure à celle de la doxorubicine seule, à concentration égale. Des nanoparticules témoins, sans principe actif fixé, ne présentent aucune toxicité.

On constate par ailleurs que ces mêmes nanoparticules peuvent être aisément phagocytées et aussi subir rapidement un processus d'endocytose par des cellules très différenciées comme par exemple les hépatocytes animaux ou humains.

L'efficacité de l'application des nanoparticules en thérapeutique est démontrée par l'utilisation des nanoparticules décrites ci-dessus couplées à la doxorubicine (1mg de doxorubicine par ml de suspension de nanoparticules à 2,5% de copolymère), administrées par voie intravasculaire à un patient atteint d'un hépatocarcinome. Après 6 administrations de 90ml de suspension stérile de composition ci-dessus, espacées chacunes de 4 semaines, on observe une nette amélioration de l'état du patient, confirmée par les examens biologiques et les explorations cliniques et radiologiques.

## Revendications

1. Nanoparticules à base de polymère ou copolymère méthacrylique, de forme sphérique, de diamètre moyen compris entre 100 et 600nm, caractérisées en ce qu'elles sont de distribution homogène (système monodispersé), et présentent en surface, ou dans les pores ouverts en surface, des groupements réactionnels hydroxyle et/ou carboxyle.

2. Nanoparticules à base de polymère ou copolymère méthacrylique selon la revendication 1, caractérisées en ce qu'elles comprennent au moins une unité récurrente de formule générale (I) ci-après:

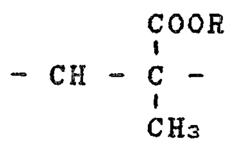

dans laquelle R représente un atome d'hydrogène ou un groupe alkyle linéaire ou ramifié de 1 à 6 atomes de carbone, ou un groupe alkyle substitué par un groupe hydroxy, carboxy ou ester, comportant le cas échéant une ou plusieurs doubles liaisons éthyléniques, l'une au moins des unités récurrentes comportant au moins un groupe carboxyle et/ou hydroxyle libre.

3. Nanoparticules selon la revendication 2, caractérisées en ce que R est un atome d'hydrogène ou un groupe méthyle, éthyle, n-butyle, hydroxy-2 propyle ou isopropényl carboxylate.

4. Nanoparticules selon l'une quelconque des revendications 1 à 3, caractérisées en ce que leur diamètre moyen est compris entre 200 et 400nm.

5. Procédé de préparation de nanoparticules selon la revendication 1, caractérisé en ce qu'on effectue une polymérisation en émulsion à partir d'au moins un monomère méthacrylique de formule générale (II) ci-après:

$$CH_2 = \underset{\substack{| \\ CH_3}}{C} - COOR$$

dans laquelle R représente un atome d'hydrogène ou un groupe alkyle linéaire ou ramifié de 1 à 6 atomes de carbone, ou un groupe alkyle substitué par un groupe hydroxy, carboxy ou ester, comportant le cas échéant une ou plusieurs doubles liaisons éthyléniques, l'un au moins des monomères comportant un groupe R alkyle substitué par un groupe hydroxy et/ou carboxy et/ou ester, en effectuant la polymérisation en phase aqueuse, en présence d'un initiateur de radicaux libres, sous agitation, à une température comprise entre 60 et 120 °C.

6. Procédé selon la revendication 5, caractérisé en ce que la température de polymérisation est comprise entre 80 et 90 °C.

7. Procédé selon la revendication 5, caractérisé en ce que l'initiateur de radicaux libres est choisi parmi le peroxodisulfate de potassium, le persulfate d'ammonium et le persulfate de potassium.

8. Procédé selon l'une quelconque des revendications 5 à 7, caractérisé en ce que le monomère est choisi parmi l'acide méthacrylique, le méthacrylate de méthyle ou de n-butyle, le méthacrylate, d'hydroxy-2 propyle et le diméthacrylate d'éthylène glycol, isolément ou en combinaison.

9. Procédé selon la revendication 5, caractérisé en ce que la polymérisation s'effectue en présence d'un agent tensio-actif.

10. Vecteur de médicament, caractérisé en ce qu'il comprend des nanoparticules selon l'une quelconque des revendications 1 à 4 sur lesquelles est fixée au moins une molécule pharmacologiquement active.

11. Vecteur de médicament selon la revendication 10, caractérisé en ce qu'il comporte en outre au moins un anticorps monoclonal fixé sur les nanoparticules par liaison covalente.

12. Vecteur de médicament selon l'une quelconque des revendications 10 et 11, caractérisé en ce que la molécule pharmacologiquement active est choisie parmi la doxorubicine, la bléomycine et la daunorubicine.

13. Réactif de diagnostic médical, caractérisé en ce qu'il comprend des nanoparticules selon l'une quelconque des revendications 1 à 4 sur lesquelles est fixée au moins une molécule bioréactive ou immunoréactive.

14. Produit de contraste pour imagerie médicale par RMN, caractérisé en ce qu'il comprend des nanoparticules selon l'une quelconque des revendications 1 à 4, sur lesquelles est fixé au moins un composé choisi parmi un oxyde de fer, un sel de manganèse et un dérivé de gadolinium.

## Claims

1. Nanoparticles on the basis Of methacrylic polymer or copolymer, of spherical form, having an average diameter of between 100 and 600 nm, characterised in that they are of homogenous distribution (monodispersed system) and have at the surface, or in the pores open at the surface, reactive hydroxyl and/or carboxyl groups.

2. Nanoparticles an the basis of methacrylic polymer or copolymer according to Claim 1, characterised in that they comprise at least one recurring unit of the general formula (I) below:

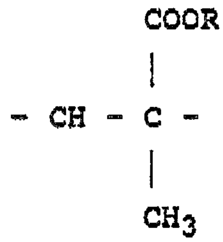

in which R represents a hydrogen atom or a straight or branched alkyl group having 1 to 6 carbon atoms, or an alkyl group substituted by a hydroxy, carboxy or ester group, comprising if necessary one or more double ethylene bonds, at least one of the recurring units comprising at least one free carboxyl and/or hydroxyl group.

**3.** Nanoparticles according to Claim 2, characterised in that R is a hydrogen atom or a methyl, ethyl, n-butyl, 2-hydroxypropyl or isopropenyl carboxylate group.

**4.** Nanoparticles according to any one of Claims 1 to 3, characterised in that their average diameter is between 200 and 400 nm.

**5.** A method for preparing nanoparticles according to Claim 1, characterised in that emulsion polymerisation is carried out starting from at least one methacrylic monomer of the general formula (II) below:

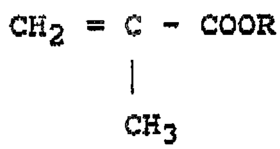

in which R represents a hydrogen atom or a straight or branched alkyl group having 1 to 6 carbon atoms, or an alkyl group substituted by a hydroxy, carboxy or ester group, comprising if necessary one or more double ethylene bonds, at least one of the monomers comprising an alkyl group R substituted by a hydroxy and/or carboxy and/or ester group, by performing the polymerisation in the aqueous phase in the presence of a free radical initiator, with stirring, at a temperature of between 60 and 120°C.

**6.** A method according to Claim 5, characterised in that the polymerisation temperature lies between 80 and 90°C.

**7.** A method according to Claim 5, characterised in that the free radical initiator is selected from potassium peroxodisulphate, ammonium persulphate and potassium persulphate.

**8.** A method according to any one of Claims 5 to 7, characterised in that the monomer is selected from methacrylic acid, methyl methacrylate or n-butyl methacrylate, 2-hydroxypropyl methacrylate and ethylene glycol dimethacrylate, singly or in combination.

**9.** A method according to Claim 5, characterised in that the polymerisation is carried out in the presence of a surface-active agent.

**10.** A carrier for medicaments, characterised in that it comprises nanoparticles according to any one of Claims 1 to 4 to which is attached at least one pharmacologically active molecule.

**11.** A vector for medicines according to Claim 10, characterised in that it further comprises at least one monoclonal antibody attached to the nanoparticles by a covalent bond.

12. A vector for medicines according to any one of Claims 10 and 11, characterised in that the pharmacologically active molecule is selected from doxorubicin, bleomycin and daunorubicin.

13. A reagent for medical diagnostics, characterised in that it comprises nanoparticles according to any one of Claims 1 to 4 to which is attached at least one bioreactive or immunoreactive molecule.

14. A contrast product for medical imaging by NMR, characterised in that it comprises nanoparticles according to any one of Claims 1 to 4 to which is attached at least one compound selected from an iron oxide, a manganese salt and a gadolinium derivative.

**Ansprüche**

1. Nanopartikel auf Basis eines Methacrylpolymeren oder -copolymeren mit Kugelform und einem mittleren Durchmesser zwischen 100 und 600 nm, dadurch gekennzeichnet, daß sie eine homogene Verteilung aufweisen (ein monodisperses System darstellen) und an der Oberfläche oder in den an der Oberfläche offenen Poren reaktionsfähige Hydroxyl- und/oder Carboxylgruppen aufweisen.

2. Nanopartikel auf Basis eines Methacrylpolymeren oder -copolymeren nach Anspruch 1, dadurch gekennzeichnet, daß sie mindestens eine wiederkehrende Einheit der nachstehend angegebenen allgemeinen Formel (I) aufweisen:

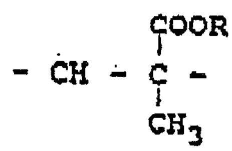

worin R ein Wasserstoffatom oder eine lineare oder verzweigte Alkylgruppe mit 1 bis 6 Kohlenstoffatomen oder eine Alkylgruppe, die durch eine Hydroxy-, Carboxy- oder Estergruppe substituiert ist, die gegebenenfalls eine oder mehrere ethylenische Doppelbindungen aufweist, darstellt, wobei mindestens eine der wiederkehrenden Einheiten mindestens eine freie Carboxyl-und/oder Hydroxylgruppe aufweist.

3. Nanopartikel nach Anspruch 2, dadurch gekennzeichnet, daß R ein Wasserstoffatom oder eine Methyl-, Ethyl-, n-Butyl-, 2-Hydroxypropyl- oder Isopropenylcarboxylatgruppe darstellt.

4. Nanopartikel nach einem der Ansprüche 1 bis 3, dadurch gekennzeichnet, daß ihr mittlerer Durchmesser zwischen 200 und 400 nm liegt.

5. Verfahren zur Herstellung der Nanopartikel nach Anspruch 1, dadurch gekennzeichnet, daß man eine Polymerisation in Emulsion durchführt, wobei man ausgeht von mindestens einem Methacrylmonomeren der nachstehend angegebenen allgemeinen Formel (II)

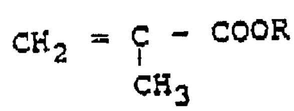

worin R ein Wasserstoffatom oder eine lineare oder verzweigte Alkylgruppe mit 1 bis 6 Kohlenstoffatomen oder eine Alkylgruppe darstellt, die durch eine Hydroxy-, Carboxy- oder Estergruppe substituiert ist, die gegebenenfalls eine oder mehrere ethylenische Doppelbindungen aufweist, wobei mindestens eines der Monomeren eine Alkylgruppe R aufweist, die durch eine Hydroxy- und/oder Carboxy- und/oder Estergruppe substituiert ist, wobei man die Polymerisation in wäßriger Phase in Gegenwart eines Freiradikal-Initiators unter Rühren bei einer Temperatur zwischen 60 und 120 °C durchführt.

6. Verfahren nach Anspruch 5, dadurch gekennzeichnet, daß die Polymerisationstemperatur zwischen 80 und 90 °C liegt.

7. Verfahren nach Anspruch 5, dadurch gekennzeichnet, daß der Freiradikal-Initiator ausgewählt wird aus Kaliumperoxodisulfat, Ammoniumpersulfat und Kaliumpersulfat.

8. Verfahren nach einem der Ansprüche 5 bis 7, dadurch gekennzeichnet, daß das Monomere ausgewählt wird aus Methacrylsäure, Methylmethacrylat oder n-Butylmethacrylat, 2-Hydroxypropylmethacrylat und Ethylenglycoldimethacrylat, einzeln oder in Kombination.

9. Verfahren nach Anspruch 5, dadurch gekennzeichnet, daß die Polymerisation in Gegenwart eines oberflächenaktiven Agens durchgeführt wird.

10. Arzneimittelvektor, dadurch gekennzeichnet, daß er Nanopartikel nach einem der Ansprüche 1 bis 4 umfaßt, auf denen mindestens ein pharmakologisch aktives Molekül fixiert ist.

11. Arzneimittelvektor nach Anspruch 10, dadurch gekennzeichnet, daß er außerdem mindestens einen monoklonalen Antikörper umfaßt, der an den Nanopartikeln durch covalente Bindung fixiert ist.

12. Arzneimittelvektor nach einem der Ansprüche 10 und 11, dadurch gekennzeichnet, daß das pharmakologisch aktive Molekül ausgewählt wird aus Doxorubicin, Bleomycin und Daunorubicin.

13. Medizinisches diagnostisches Reagens, dadurch gekennzeichnet, daß es umfaßt Nanopartikel nach einem der Ansprüche 1 bis 4, auf denen mindestens ein bioreaktives oder immunoreaktives Molekül fixiert ist.

14. Kontrastmittel zur Erzeugung eines medizinischen Bildes durch NMR, dadurch gekennzeichnet, daß es Nanopartikel nach einem der Ansprüche 1 bis 4 umfaßt, auf denen mindestens eine Verbindung, ausgewählt aus einem Eisenoxid, einem Mangansalz und einem Gadoliniumderivat, fixiert ist.